(19) Europäisches Patentamt / European Patent Office / Office européen des brevets

(11) **EP 4 197 623 A1**

(12) **EUROPÄISCHE PATENTANMELDUNG**

(43) Veröffentlichungstag:
**21.06.2023 Patentblatt 2023/25**

(21) Anmeldenummer: **21215862.0**

(22) Anmeldetag: **20.12.2021**

(51) Internationale Patentklassifikation (IPC):
**B01D 61/02** (2006.01)    **B01D 69/12** (2006.01)
**B01D 71/52** (2006.01)    **B01J 31/00** (2006.01)
**B01J 31/40** (2006.01)

(52) Gemeinsame Patentklassifikation (CPC):
**B01D 61/027; B01D 69/12; B01D 71/52;
B01J 31/4061;** B01J 2531/824

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH
45128 Essen (DE)**

(72) Erfinder:
- **KUCMIERCZYK, Peter
  44628 Herne (DE)**
- **FRIDAG, Dirk
  45721 Haltern am See (DE)**
- **SCHÄPERTÖNS, Marc
  45657 Recklinghausen (DE)**
- **GLUTH, Frederik
  45472 Mülheim an der Ruhr (DE)**
- **DREIMANN, Jens Martin
  44141 Dortmund (DE)**
- **FRANKE, Robert
  45772 Marl (DE)**
- **KNOSSALLA, Johannes
  46514 Gahlen (DE)**
- **SALE, Anna Chiara
  45657 Recklinghausen (DE)**
- **MARKOVIC, Ana
  45721 Haltern am See (DE)**
- **BRÄCHER, Alexander
  45721 Haltern am See (DE)**

(74) Vertreter: **Evonik Patent Association
c/o Evonik Industries AG
IP Management
Bau 1042A/PB 15
Paul-Baumann-Straße 1
45772 Marl (DE)**

(54) **VERFAHREN ZUR HERSTELLUNG EINES ESTERS UND ABTRENNUNG DES HOMOGENEN KATALYSATORSYSTEMS UNTER VERWENDUNG EINER MEMBRAN UMFASSEND EINE TRENNAKTIVE SCHICHT AUS PAEK UND EINER UNTERSTRUKTUR, DIE PAEK UMFASST**

(57)    Gegenstand der Erfindung ist ein Verfahren zur Herstellung eines Esters durch Alkoxycarbonylierung eines C2- bis C16-Kohlenwasserstoffs mit mindestens einer Mehrfachbindung, vorzugsweise mit mindestens einer olefinischen Doppelbindung, bei dem das eingesetzte homogene Katalysatorsystem mittels Membrantrennung unter Verwendung einer Membran umfassend eine trennaktive Schicht aus PAEK und einer Unterstruktur, die PAEK umfasst, aus dem Produktgemisch abgetrennt und zur Reaktionszone zurückgeführt wird. In einer Weiterbildung der vorliegenden Erfindung wird der so gebildete Ester durch Umesterung zu einem anderen Ester umgesetzt.

EP 4 197 623 A1

**Beschreibung**

Die vorliegende Erfindung betrifft ein Verfahren zur Herstellung eines Esters durch Alkoxycarbonylierung eines C2- bis C16-Kohlenwasserstoffs mit mindestens einer Mehrfachbindung, vorzugsweise mit mindestens einer olefinischen Doppelbindung, bei dem das eingesetzte homogene Katalysatorsystem mittels Membrantrennung unter Verwendung einer Membran umfassend eine trennaktive Schicht aus PAEK und einer Unterstruktur, die PAEK umfasst, aus dem Produktgemisch abgetrennt und zur Reaktionszone zurückgeführt wird. In einer Weiterbildung der vorliegenden Erfindung wird der so gebildete Ester durch Umesterung zu einem anderen Ester umgesetzt.

Die Alkoxycarbonylierung ist die Umsetzung eines Kohlenwasserstoffs, der mindestens eine Mehrfachbindung, vorzugsweise mindestens eine olefinische Doppelbindung aufweist, mit Kohlenmonoxid und einem Alkohol zu den entsprechenden Estern. Dabei werden üblicherweise Metall-Ligand-Komplexe als Katalysator eingesetzt, die homogen gelöst im Reaktionsgemisch und damit auch im Produktgemisch vorliegen. Um den gewünschten Ester aus dem Produktgemisch isolieren zu können, ist es von Vorteil zuvor zumindest den Großteil an homogen gelösten Katalysator aus dem Produktgemisch zu entfernen. Auf der anderen Seite sind die Kosten für die eingesetzten Katalysatoren vergleichsweise hoch, weshalb der Katalysator auch schon aus wirtschaftlichen Gründen zurückgewonnen werden sollte.

Für die Abtrennung des homogen gelösten Katalysators vom restlichen Reaktionsgemisch sind verschiedene Verfahren bekannt. Die WO 2013/107902 A1 offenbart beispielsweise ein Verfahren, bei dem der homogen gelöste Katalysator nach Alkoxycarbonylierung mittels einer Membran abgetrennt wird, die für Moleküle ab einem Molekülgewicht von 1 kDa undurchlässig ist, wodurch der Katalysator zurückgehalten und im Retentat angereichert wird. Gemäß der WO 2013/107902 A1 sind auf Polyimiden oder Polydimethylsiloxan basierende Membranmaterialien bevorzugt.

Die in der WO 2013/107902 A1 offenbarten Materialien weisen aber den Nachteil auf, dass sie oft keine ausreichende Langzeitstabilität vor allem gegenüber Säuren aufweisen, welche als CoKatalysatoren typischerweise Teil des Katalysatorsystems sind, und bei mehrfacher Beanspruchung instabil werden können. Weiterhin weisen diese Membranmaterialien keinen nennenswerten Rückhalt für den eingesetzten Kohlenwasserstoff oder das Lösungsmittel auf, welches gleichzeitig Edukt bei der Alkoxycarbonylierung sein kann, beispielsweise der eingesetzte Alkohol. Weder Alkohol noch Kohlenwasserstoff werden im Retentat angereichert. Dadurch müssen nachgeschaltete Aufarbeitungs- und/oder Aufreinigungsschritte, wie nachfolgende Destillationsschritte, aufwendiger betrieben werden, um das Lösungsmittel bzw. die Edukte vom Produkt abzutrennen.

Die daraus resultierende Aufgabe der vorliegenden Erfindung war die Bereitstellung eines Verfahrens zur Alkoxycarbonylierung, bei der der homogene Katalysator unter Verwendung eines säurestabilen Membranmaterials, welches auch über einen längeren Zeitraum eingesetzt werden kann, abgetrennt wird. Weiterhin war die Aufgabe ein Alkoxycarbonylierungsverfahren bereitzustellen, wobei bei der Membranabtrennung bevorzugt die gebildeten Produkte durchgelassen und zumindest für einen Teil der eingesetzten Edukte, insbesondere den Alkohol, zumindest teilweise zurückgehalten werden. Eine weitere Aufgabe der vorliegenden Erfindung war die Bereitstellung eines neuartigen Membranmaterials für die Abtrennung des homogen gelösten Katalysatorsystems.

Die zugrundeliegende Aufgabe der vorliegenden Erfindung konnte durch Verfahren nach Anspruch 1 gelöst werden. Bevorzugte Ausführungsformen sind in den Unteransprüchen angegeben.

Erfindungsgemäß umfasst das Verfahren zur Herstellung eines Esters durch Carbonylierung eines C2- bis C16-Kohlenwasserstoffs die folgenden Schritte:

a) Umsetzen (Carbonylieren) eines C2- bis C16-Kohlenwasserstoffs mit mindestens einer olefinischen Doppelbindung mit Kohlenmonoxid und mit einem Alkohol, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher, wenn es sich um einen Monoalkohol handelt, in einem Molverhältnis zum C2- bis C16-Kohlenwasserstoff (Alkohol: C2- bis C16-Kohlenwasserstoff) von 2 bis 20 eingesetzt wird oder welcher, wenn es sich um einen Polyalkohol handelt, in einem Molverhältnis zum eingesetzten Kohlenwasserstoff (Kohlenwasserstoff: Polyalkohol) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches, welches zumindest den durch die Umsetzung gebildeten Ester, das homogene Katalysatorsystem, Leichtsieder, und/oder Hochsieder und nicht umgesetzte Alkohole umfasst;

b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol, vorzugsweise nicht umgesetzter Alkohol im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht, und Rückführung des Retentats in die Reaktionszone;

c) Aufarbeitung des Permeat mittels eines oder mehrerer Trennschritte, ausgewählt aus thermischer Trennung, Extraktion, Kristallisation oder Membrantrennung, vorzugsweise mittels eines oder mehrerer Destillationsschritte, zur Abtrennung des in Schritt a) gebildeten Esters und zur Abtrennung des nicht umgesetzten C2- bis C16-Kohlenwasserstoffs mit mindestens einer olefinischen Doppelbindung und/oder des nicht umgesetzten Alkohols und Rückführung des nicht umgesetzten C2- bis C16-Kohlenwasserstoffs mit mindestens einer olefinischen Doppelbindung und/oder des nicht umgesetzten Alkohols in die Reaktionszone von Schritt a).

Thermische Trennung im Sinne der vorliegenden Erfindung meint ein Trennverfahren, bei dem die Trennung anhand des Siedepunktes erfolgt.

Die bei der Umsetzung in Schritt a) eingesetzten Kohlenwasserstoffe müssen mindestens eine Mehrfachbindung aufweisen. Bevorzugt sind Kohlenwasserstoffe mit mindestens einer olefinischen Doppelbindung, besonders bevorzugt sind Kohlenwasserstoffe mit einer olefinischen Doppelbindung. Grundsätzlich ist die Anzahl der Kohlenstoffatome von Verbindung, die mindestens eine Mehrfachbindung, vorzugsweise mindestens eine olefinische Doppelbindung aufweisen, nicht begrenzt. Technische relevant sind aber nur die Carbonylierung von C2- bis C16-Kohlenwasserstoffen mit mindestens einer Mehrfachbindung, vorzugsweise mindestens einer olefinischen Doppelbindung. In einer bevorzugten Ausführungsform der vorliegenden Erfindung können C3- bis C12-Kohlenwasserstoffe, vorzugsweise mit mindestens einer olefinischen Doppelbindung eingesetzt werden. Darunter fallen insbesondere n-Alkene, iso-Alkene, Cycloalkene und aromatische Alkene mit 2 bis 16 Kohlenstoffatomen, vorzugsweise 3 bis 12 Kohlenstoffatomen.

Die vorgenannt beschriebenen Kohlenwasserstoffe können zusätzlich zu der mindestens einen olefinischen Doppelbindungen eine oder mehrere weitere funktionelle Gruppen enthalten. Beispiele für geeigente funktionelle Gruppen sind Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl-, Amino-, Ether-, Thioether-, Aryl-, Heteroaryl- oder Silylgruppen und/oder Halogensubstituenten.

Besonders bevorzugte Kohlenwasserstoffe, die in Schritt a) des erfindungsgemäßen Verfahrens eingesetzt werden, weisen nur eine olefinische Doppelbindung auf, insbesondere n-Alkene und iso-Alkene mit 2 bis 16 Kohlenstoffatomen, vorzugsweise 3 bis 12 Kohlenstoffatomen. Die eingesetzten Kohlenwasserstoffe sind vorzugsweise unsubstituiert.

Die eingesetzten und vorher beschriebenen Kohlenwasserstoffe mit olefinischer Doppelbindung werden erfindungsgemäß mit Kohlenmonoxid (CO) und einem Alkohol zum entsprechenden Ester in Schritt a) umgesetzt. Das Kohlenmonoxid kann dabei direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt a) eingesetzte Alkohol ist ein Mono- oder Polyalkohol (zwei oder mehr OH-Gruppe) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono-und/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Der in Schritt a) eingesetzte Alkohol wird, wenn es sich um einen Monoalkohol handelt, in einem Molverhältnis zum eingesetzten Kohlenwasserstoff (Monoalkohol : Kohlenwasserstoff) von 2 bis 20, vorzugsweise von 3 bis 10 und besonders bevorzugt von 4 bis 6 eingesetzt. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der in Schritt a) eingesetzte Alkohol wird, wenn es sich um einen Polyalkohol handelt, in einem Molverhältnis zum eingesetzten Kohlenwasserstoff (Kohlenwasserstoff : Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt.

Die erfindungsgemäße Umsetzung in Schritt a) wird in Gegenwart eines homogenen Katalysatorsystems durchgeführt, welches zumindest ein Metall aus der Gruppe 8 bis 10 des Periodensystems der Elemente (PSE) oder eine Verbindung davon, einen phosphorhaltigen Liganden und eine Säure als Co-Katalysator umfasst.

Das Metall aus der Gruppe 8 bis 10 des PSE ist vorzugsweise Palladium oder Platin, besonders bevorzugt Platin. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [$PdCl_2$], Palladium(II)-Acetylacetonat [$Pd(acac)_2$], Palladium(II)-Acetat [$Pd(OAc)_2$], Dichloro-(1,5-cyclooctadien)palladium(II) [$Pd(cod)Cl_2$], Bis(dibenzylideneaceton)palladium(0) [$Pd(dba)_2$], Tris(dibenzylideneaceton)dipalladium(0) [$Pd_2(dba)_3$] Bis(acetonitril)-dichloropalladium(II) [$Pd(CH_3CN)_2Cl_2$], Palladium(cinnamyl)-dichlorid [$Pd(cinnamyl)Cl_2$]. Vorzugsweise kommen die Verbindungen [$Pd(acac)_2$] oder [$Pd(OAc)_2$] zum Einsatz. Die Metallkonzentration von Palladium in Schritt a) beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt a) 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Lewis Säuren mit einem LAU-Wert von mehr als 25, vorzugsweise mit einem LAU-Wert von 29. Der LAU-Wert ist eine neue Methode zur Bestimmung der Stärke von Lewis-Säuren (JR Gaffen et al., Chem, Vol. 5, Issue 6, S. 1567-1583). Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von $pKs \leq 5$, besonders bevorzugt eine Säurestärke von $pKs \leq 3$. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Umsetzung bzw. Carbonylierung des eingesetzten Kohlenwasserstoffs mit olefinischer Doppelbindung in Schritt a) wird vorzugsweise bei einer Temperatur von 25 bis 140 °C, weiterhin bevorzugt bei einer Temperatur von 70 bis 130 °C und besonders bevorzugt bei einer Temperatur von 80 bis 120 °C durchgeführt. Der Druck in Schritt a) kann zwischen 5 und 60 bar, vorzugsweise zwischen 10 und 40 bar, besonders bevorzugt zwischen 15 und 30 bar betragen.

Die beschriebene Umsetzung in Schritt a) findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Umsetzung in Schritt a) wird ein flüssiges Produktgemisch erhalten, die zumindest den durch die Umsetzung gebildeten Ester, das homogene Katalysatorsystem, nicht umgesetzte Alkohole A, und ggf. weitere Komponenten wie Leichtsieder, beispielsweise leichtsiedende Nebenprodukte wie Ether, und/oder Hochsieder und/oder nicht umgesetzte Kohlenwasserstoffe umfasst. Das Produktgemisch wird dann der nachfolgenden Membrantrennung in Schritt b) zugeführt. Bei der Umsetzung in Schritt a) kann zudem ein Abgas, welches zumindest aus unreaktiven Verunreinigungen wie Stickstoff, Wasserstoff, Alkanen und leichtsiedenden Nebenprodukten (beispielsweise die bereits genannten Ether) aus der Reaktionszone entnommen werden. Die Verunreinigungen und leichtsiedenden Nebenprodukte könnten sich akkumulieren und den Partialdruck des Reaktionsgases (CO) auf lange Sicht erniedrigen, wodurch die Reaktion verlangsamt werden könnte.

Im nachfolgenden Schritt b) wird das flüssige Produktgemisch einer Membrantrennung zugeführt, um das homogene Katalysatorsystem vom Produktgemisch abzutrennen. Durch das erfindungsgemäße Membranmaterial werden das homogene Katalysatorsystem und nicht umgesetzter Kohlenwasserstoff und/oder nicht umgesetzter Alkohol im Retentat angereichert, während der in Schritt a) gebildete Ester im Permeat angereichert wird. Das Permeat, welches an gebildeten Ester angereichert ist, wird dann zum nachfolgenden Schritt c) geführt. Das Retentat, welches das angereicherte homogene Katalysatorsystem umfasst, wird dann in die Reaktionszone zurückgeführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen enthalten kann, die durch die eingesetzten Kohlenwasserstoffströme eingetragen werden, wie beispielsweise Spuren von Wasser oder Stickstoff, entnommen werden, um eine Akkumulation in der oder den Reaktionszonen zu vermeiden. Die Rückführung des Retentats sorgt dafür, dass das bei der Membrantrennung im Retentat angereicherte Katalysatorsystem zur Reaktion zurückgeführt wird. Katalysatorverluste durch im Permeat befindliche Reste an Katalysator, die durch eine Membran durchtreten, sind meistens nicht ganz zu vermeiden, aber die erwähnte Verringerung der Verluste führt dazu, dass weniger Katalysator durch Zuführung von frischem Katalysator ersetzt werden muss.

Die Membrantrennung beruht auf der Semipermeabilität der dabei eingesetzten Membran, welche für bestimmte Stoffe durchlässig und für andere Stoffe undurchlässig oder deutlich weniger durchlässig ist. Die in Schritt b) des erfindungsgemäßen Verfahrens eingesetzte Membran umfasst eine auf einer Unterstruktur aufgebrachte trennaktive Schicht, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht. In einer bevorzugten Ausführungsform der vorliegenden Erfindung besteht die bei der Membrantrennung in Schritt b) eingesetzte Membran aus einer auf einer Unterstruktur aufgebrachte trennaktive Schicht, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht.

Die Permeanz (Engl.: permeance) ist das wesentliche Unterscheidungsmerkmal zwischen der trennaktiven Schicht, an der bzw. in der die gewünschte Trennung abläuft, und der Unterstruktur, die keine Trennwirkung entfalten soll. Mit Permeanz ist im Rahmen der vorliegenden Erfindung der Druck-normalisierte Fluss (Engl.: pressure-normalized flux) gemeint. Für Membranen, welche in der Gastrennung eingesetzt werden, ist für Permeanz eine häufig genutzte Einheit gpu (Engl.: Gas Permeation Units). Für Membranen im Bereich der organophilen Nanofiltration (Engl.: Organic Solvent Nanofiltration) hat sich die Einheit $L\ m^{-2}\ h^{-1}\ bar^{-1}$ bewährt. Die Permeanz ist von der Permeabilität zu unterscheiden, da Permeabilität neben Druck auch in Bezug auf die Schichtdicke normalisiert (Engl.: thickness-normalized) ist. Es ist erfindungsgemäß bevorzugt, wenn die Unterstruktur keinen nennenswerten Transportwiderstand gegenüber der zu trennenden Mischung bietet und daher eine mindestens zweifach höhere Permeanz, vorzugsweise eine mindestens 10-fach höhere Permeanz, besonders bevorzugt eine mindestens 100-fach höhere Permeanz aufweist als die trennaktive Schicht.

Zur Messung der Permeanz von Unterstrukturen und trennaktiven Schichten eignet sich z.B. eine Messung der Luftdurchlässigkeit nach DIN EN ISO 9073-15 (August 2008). Die Messung von Luftdurchlässigkeit nach diesem Verfahren findet aufgrund der hohen Durchlässigkeit bei Differenzdrücken von nur etwa 2 mbar statt. Gängige Membranen für den Einsatz in der organophilen Nanofiltration haben in Lösemitteln wie Aceton oder Toluol eine Permeanz im Bereich von etwa 0,1 bis 10 $L\ m^{-2}\ h^{-1}\ bar^{-1}$. Nanofiltrationsmembranen werden daher typischerweise bei Differenzdrücken zwischen 20 bis 40 bar (20,000 bis 40,000 mbar) im Temperaturbereich leicht über Raumtemperatur (z.B. 30 bis 40 °C) vermessen, entweder als Flachmembranen mit aktiven Membranflächen von ca. 80 $cm^2$ oder als kleine Spiralwickelelemente mit aktiven Membranflächen von 0,1 bis 2 $m^2$. Die Permeanz einer PEEK-Membran im System 43 : 57 w/w Methanol: Methyloctanoat bei 20 bar Transmembrandruck und 40 °C ist zum Beispiel etwa 0,2 $L\ m^{-2}\ h^{-1}\ bar^{-1}$.

Die in Schritt b) eingesetzte Membran umfassend oder bestehend aus trennaktive(r) Schicht und Unterstruktur ist vorzugsweise säurestabil. Der Begriff "säurestabil" meint im Rahmen der vorliegenden Erfindung, dass die Membran mindestens 300 h in Anwesenheit der Säure des Katalysatorsystems, insbesondere einer Brönsted-Säure mit einem $pKs \leq 5$, besonders bevorzugt mit einem $pKs \leq 3$ oder einer Lewis-Säure mit einem LAU-Wert von mehr als 25, vorzugsweise mit einem LAU-Wert von 29, stabil ist und nicht zerstört wird, d. h. die trennaktive Schicht und die Unterstruktur bleiben intakt.

Die trennaktive Schicht der Membran besteht aus einem Polyaryletherketon (PAEK). PAEK zeichnet sich dadurch aus, dass innerhalb der Wiederholungseinheit Arylgruppen über mindestens eine Etherfunktionalität und mindestens eine Ketonfunktionalität verknüpft sind. Eine erfindungsgemäß bevorzugte trennaktive Schicht besteht aus einem Polyetheretherketon (PEEK). Es hat sich gezeigt, dass diese Materialien chemisch besonders stabil sind, insbesondere auch gegenüber Säuren. Als trennaktive Schicht wird weiterhin bevorzugt ein Polyaryletherketon (PAEK) mit einem Sulfonierungsgrad von weniger als 20%, besonders bevorzugt von weniger als 10%, besonders bevorzugt Polyetheretherketon (PEEK) mit einem Sulfonierungsgrad von weniger als 20%, besonders bevorzugt von weniger als 10% eingesetzt. Der Sulfonierungsgrad kann darüber bestimmt werden, dass zunächst die Gehalte von Kohlenstoff (C) und Schwefel (S) bestimmt werden. Dies kann beispielsweise mittels Elementaranalyse oder für Schwefel auch mittels Titration erfolgen. Der Sulfonierungsgrad kann dann anhand der folgenden Formel berechnet werden:

$$\text{Sulfonierungsgrad (\%)} = \frac{\text{experimentell bestimmtes Verhältnis von S zu C in der Probe}}{\text{theor. Verhältnis von S zu C bei 100\%iger Sulfonierung}} * 100$$

Es ist möglich, dass das für die Herstellung eingesetzte PAEK-Rohpolymer schon einen geringen Anteil an Schwefel enthalten kann, was einem Sulfonierungsgrad von bis zu 3 % entsprechen kann. Dieser geringe Anteil an Schwefel kann z. B. aus dem Einsatz des Hochtemperaturlösemittels Diphenylsulfon bei der Herstellung resultieren und entspricht nicht zwangsläufig einer Sulfonierung. Es kann daher in bestimmten Fällen notwendig sein, den Schwefelgehalt des Rohpolymers vor Verarbeitung zur Membran kennen und zu berücksichtigen, um den Sulfonierungsgrad der resultierenden Membran korrekt zu bestimmen.

Die Unterstruktur der Membran enthält erfindungsgemäß ein Polyaryletherketon (PAEK), vorzugsweise aus einem Polyetheretherketon (PEEK). Im Gegensatz zur trennaktiven Schicht kann die Unterstruktur auch andere Komponenten, beispielsweise andere Polymere enthalten. Es ist erfindungsgemäß jedoch bevorzugt, wenn das Polyaryletherketon (PAEK), vorzugsweise das Polyetheretherketon (PEEK) die Hauptkomponente ist, d. h. die Unterstruktur zu mehr als 50 Gew.- % aus dem Polyaryletherketon (PAEK) besteht, vorzugsweise dem Polyetheretherketon (PEEK). In einer bevorzugten Ausführungsform der vorliegenden Erfindung besteht die Unterstruktur zu mindestens 90 Gew.-% aus dem Polyaryletherketon (PAEK), vorzugsweise dem Polyetheretherketon (PEEK), weiterhin bevorzugt zu mindestens 99 Gew.-% aus dem Polyaryletherketon (PAEK), vorzugsweise dem Polyetheretherketon (PEEK). In einer besonders bevorzugten Ausführungsform besteht die Unterstruktur ausschließlich aus dem Polyaryletherketon (PAEK), vorzugsweise dem Polyetheretherketon (PEEK). Es hat sich gezeigt, dass diese Materialien chemisch besonders stabil sind, insbesondere auch gegenüber Säuren.

Die Unterstruktur kann dabei aus mehreren Schichten bestehen, wobei die einzelnen Schichten aus unterschiedlichen Materialien, z.B. unterschiedlichen Polymeren, Mischungen aus unterschiedlichen Materialien, oder Verbundsystemen bestehen können, wobei die vorgenannten bevorzugten Mindestmengen an Polyaryletherketon (PAEK), vorzugsweise dem Polyetheretherketon (PEEK) vorhanden sein müssen. Im einfachsten Fall besteht die Unterstruktur aus einer einzigen Schicht. Ein Beispiel dafür sind Vliesstoffe, insbesondere des Typs Viledon® Novatexx 2471 (Freudenberg Filtration Technologies, Germany). Viledon® Novatexx 2471 besteht aus Mikrofasern, die im Kern aus Polypropylen (PP) und im Mantel aus Polyethylen (PE) bestehen. Die erfindungsgemäße Membran ist vorzugsweise eine Nanofiltrationsmembran, welche bei 40 °C eine Permeanz für einfache aromatische Kohlenwasserstoffe, bspw. Toluol, von 0,01 bis 10 L/(m$^2$ h bar), sowie einen Molecular Weight Cutoff (MWCO) zwischen 100 bis 2000 Da aufweist. Der MWCO ist als das Molekulargewicht einer Komponente mit einem Rückhalt durch die Membran von 90% definiert. Eine Nanofiltrationsmembran ist gemäß der vorliegenden Erfindung eine Membran, welche für Moleküle mit einem Molekülgewicht im Bereich zwischen 200 bis 2000 Da eine geeignete Trennwirkung entfaltet.

Ein Verfahren zur Herstellung der bei der Membrantrennung in Schritt b) eingesetzten Membran umfasst zumindest die folgenden Schritte:

a) Herstellen einer Polymerlösung, umfassend ein Polyaryletherketon (PAEK), vorzugsweise ein Polyetheretherketon (PEEK), und mindestens ein Lösemittel;

b) Bereitstellen einer Unterstruktur, die ein Polyaryletherketon (PAEK) enthält und die eine höhere Permeanz aufweist als die nachfolgend gebildete trennaktive Schicht;

c) Auftragen der Polymerlösung auf die Unterstruktur;

d) Durchführen einer Phaseninversion durch Eintauchen der Unterstruktur, auf die in Schritt c) die Polymerlösung aufgetragen worden ist, in einer Flüssigkeit, vorzugsweise einer wässrigen Flüssigkeit, wodurch sich die trennaktive Schicht ausbildet.

In Schritt a) des Verfahrens wird zunächst ein Polymerlösung hergestellt, die dann nachfolgend auf die Unterstruktur aufgetragen werden kann. Als Polymer wird entsprechend ein Polyaryletherketon (PAEK), vorzugsweise ein Polyetheretherketon (PEEK) oder ein Polyetherketonketon (PEKK). Hier können kommerziell verfügbare Rohstoffe eingesetzt werden, beispielsweise das Polyetheretherketon (PEEK) Vestakeep® 4000 von der Evonik Industries AG, KetaSpire® von der Solvay GmbH oder PEEK 450G™ von Victrex™. Die Rohpolymere können vor Einsatz in a) optional in der Schmelze filtriert werden, um z. B. Stippen, Gele oder andere Verunreinigungen zu entfernen. Es eignet sich z. B. eine Tiefenfiltration bei Temperaturen oberhalb von 350 °C mit Filtern mit Filterfeinheiten von 10 bis 50 μm.

Als Lösemittel kann jedes Lösemittel eingesetzt werden, in dem sich das eingesetzte Polyaryletherketon (PAEK) oder das eingesetzte Polyetheretherketon (PEEK) lösen. Bevorzugt wird im Rahmen der vorliegenden Erfindung eine Säure oder eine Mischung von zwei oder mehr Säuren eingesetzt. In einer weiterhin bevorzugten Ausführungsform der vorliegenden Erfindung wird als Lösemittel in Schritt a) Methansulfonsäure, Schwefelsäure oder eine Mischung aus Methansulfonsäure und Schwefelsäure eingesetzt. Ganz besonders bevorzugt wird als Lösemittel eine Mischung aus Methansulfonsäure und Schwefelsäure eingesetzt, wobei das Masseverhältnis Methansulfonsäure : Schwefelsäure im Bereich von 6 : 1 bis 1 : 1 liegt. Die hieraus resultierenden Polymerlösungen weisen vorzugsweise Viskositäten zwischen 1,000 mPa·s und 200,000 mPa·s auf, besonders bevorzugt zwischen 5,000 mPa·s und 100,000 mPa·s. Die Polymerlösung kann vor dem Einsatz den nachfolgenden Schritten optional filtriert werden, um z. B. Stippen, Gele oder andere Verunreinigungen zu entfernen. Die Polymerlösung kann vor dem Einsatz den nachfolgenden Schritten auch entgast werden, um Luftblasen zu entfernen.

In Schritt b) wird eine Unterstruktur bereitgestellt, die ein Polyaryletherketon (PAEK), vorzugsweise ein Polyetheretherketon (PEEK) enthält und die eine höhere Permeanz aufweist als die nachfolgend gebildete trennaktive Schicht. Die Unterstruktur weist insbesondere eine mindestens zweifach höhere Permeanz, vorzugsweise eine mindestens 10-fach höhere Permeanz, besonders bevorzugt eine mindestens 100-fach höhere Permeanz auf als die nachfolgend gebildete trennaktive Schicht. Entsprechende Unterstrukturen sind dem Fachmann geläufig. Da die Unterstruktur eine stabilisierende Funktion ausübt ist nur wichtig, dass sie eine höhere Permeanz aufweist und somit keinen wesentlichen Massentransferwiderstand ausübt. Strukturell sind die als Unterstruktur einsetzbaren Materialien also kaum beschränkt solange sie eine ausreichende mechanische Stabilität gewährleisten.

Ebenso wichtig und vorteilhaft ist, dass die Unterstruktur ein Polyaryletherketon (PAEK), vorzugsweise ein Polyetheretherketon (PEEK) enthält. Es ist erfindungsgemäß bevorzugt, dass das Polyaryletherketon (PAEK), vorzugsweise das Polyetheretherketon (PEEK) in einem hohen Anteil enthalten ist. Erfindungsgemäß bevorzugt besteht die Unterstruktur zu mindestens 90 Gew.- % aus dem Polyaryletherketon (PAEK) bzw. dem Polyetheretherketon (PEEK), vorzugsweise zu mindestens 99 Gew.-% aus dem Polyaryletherketon (PAEK) bzw. dem Polyetheretherketon (PEEK) und besonders bevorzugt ausschließlich aus dem Polyaryletherketon (PAEK) bzw. dem Polyetheretherketon (PEEK).

Im nachfolgenden Schritt c) wird dann die in Schritt a) hergestellte Polymerlösung auf die in Schritt b) bereitgestellte Unterstruktur aufgetragen. Das Auftragen kann grundsätzlich manuell mittels eines Rakels erfolgen. Industrielle Verfahren zum Auftragen entsprechender Polymerlösung sind beispielsweise der Walzenauftrag (3- bis 5-Walzensysteme), die Commabar, Schlitzdüsensysteme, z. B. Breitschlitzdüsen oder automatisierte Rakelsysteme, über die die Polymerlösung kontinuierlich aufgetragen werden kann. Diese und anderer Verfahren sind dem Fachmann aber grundsätzlich bekannt. Bevorzugt im Rahmen der vorliegenden Erfindung wird die Polymerlösung mittels eines Rakels aufgetragen, weil dies eine einfachere Handhabung und Reinigung ermöglicht. Nach dem Auftragen der Polymerlösung in Schritt c) wird die Phaseninversion in Schritt d) durchgeführt. Dazu wird die Unterstruktur, auf die in Schritt c) die Polymerlösung aufgetragen worden ist, in eine Flüssigkeit eingetaucht, wodurch sich die trennaktive Schicht ausbildet. Hierbei wird das Lösungsmittel der Polymerlösung aus dem aufgetragenen Film verdrängt und durch die Flüssigkeit verdünnt. Das gelöste Polymer ist entgegen dem Lösungsmittel nicht in der Flüssigkeit löslich, sodass dieses auf der Unterstruktur ausfällt und die trennaktive Schicht bildet. Als Flüssigkeit kann grundsätzlich jede Flüssigkeit eingesetzt werden, durch die das Lösungsmittel verdrängt und das Polymer unlöslich ist. Vorzugsweise wird als Flüssigkeit in Schritt d) eine wässrige Flüssigkeit, ein protisches Lösemittel wie Methanol oder eine Mischung dieser eingesetzt. Ferner können der Flüssigkeit lösliche Additive zugesetzt werden, welche den Vorgang der Phaseninversion günstig beeinflussen, indem sie diesen zum Beispiel verlangsamen. Entsprechende Additive sind dem Fachmann bekannt. Besonders bevorzugt wird in Schritt d) Wasser, weiterhin bevorzugt vollentsalztes Wasser eingesetzt.

Bei Herstellung von Membranen per Phaseninversion kann zwischen symmetrischen und asymmetischen (integrally skinned asymmetric) Membranen unterschieden werden. Entsteht symmetrische Membran, weist diese Membran eine durchgehend homogene Struktur auf. Entsteht eine asymmetrische Membran, weist diese Membran ausgehend von der Feedseite (Seite, auf die der Feed auf die Membran bzw. die trennaktive Schicht trifft), einen oberen Teil mit dichter Struktur und einen darunterliegenden unteren Teil mit einer fingerähnlichen Struktur auf. Im Fall der asymmetrischen Membran ist oft nur der obere dichte Teil trennaktiv. Der untere fingerähnliche Teil wird im Rahmen der vorliegenden Erfindung trotzdem der trennaktiven Schicht zugerechnet. Im Falle einer asymmetrischen Membran entstehen nämlich die trennaktive Schicht und die fingerähnliche Struktur aus derselben Polymerlösung und sind somit aus demselben Polymer, wenn sich auch aufgrund der Phaseninversionskinetik Unterschiede in Bezug auf die physische Beschaffenheit, z.B. Dichte oder Kristallinität, ergeben können.

In einer bevorzugten Ausführungsform der vorliegenden Erfindung umfasst das Verfahren zur Herstellung der Membran den folgenden zusätzlichen Schritt:

e) Waschen der in Schritt d) hergestellten Membran zur Entfernung der restlichen Säure.

Das Waschen ist deshalb optional, weil es durchaus denkbar ist, dass die Membran auch ohne vorheriges Waschen eingesetzt wird. Letztendlich kommt es auf die spezifische Anwendung an. Das Waschen dient in erster Linie der Entfernung von noch aus der Herstellung enthaltenen Säure. Sofern die in Schritt d) hergestellte Membran gemäß Schritt e) gewaschen wird, kann dazu eine wässrige Flüssigkeit oder ein anderes geeignetes Lösemittel eingesetzt werden. In einer bevorzugten Ausführungsform der vorliegenden Erfindung enthält die Membran nach dem Waschen weniger als 20% der bei der Herstellung eingesetzten Säure, vorzugsweise weniger als 10% der bei der Herstellung eingesetzten Säure, besonders bevorzugt weniger als 5% der bei der Herstellung eingesetzten Säure in Bezug auf die Gesamtmenge der Säure in der zur Herstellung der Membran eingesetzten Polymerlösung. Die wässrige Flüssigkeit für das Waschen in Schritt e) kann beispielsweise Wasser sein, welches zusätzlich Additive enthält, beispielsweise Alkohole oder Salze bzw. Elektrolyte. Bevorzugt wird zum Waschen eine wässrige Flüssigkeit, besonders bevorzugt vollentsalztes Wasser verwendet.

Nach dem Waschen kann ein Flüssigkeitsaustausch durchgeführt werden, wobei die wässrige Flüssigkeit durch eine andere Flüssigkeit, vorzugsweise ein organisches Lösemittel ausgetauscht wird. Das organische Lösemittel wird vorzugsweise aus der Gruppe, bestehend aus Methanol, Ethanol, Isopropanol, Aceton, THF und n-Hexan, ausgewählt. Bevorzugte organische Lösemittel aus dieser Gruppe sind Ethanol, Isopropanol und Aceton.

In einer besonders bevorzugten Ausführungsform der vorliegenden Erfindung umfasst das Verfahren zur Herstellung der erfindungsgemäßen Membranen den folgenden zusätzlichen Schritt:

f) Trocknen der aus Schritt d) oder Schritt e) erhaltenen Membran bei einer Temperatur im Bereich von 80 bis 180 °C, bevorzugt bei 110 bis 160 °C.

Das Trocknen kann auch ohne vorheriges Waschen stattfinden, beispielsweise dann, wenn der pH-Wert beim Einsatz der Membran nicht neutral ist. Das Trocknen der erhaltenen Membran führt dazu, dass noch vorhandene Flüssigkeit zumindest teilweise aus der Membran ausgetrieben wird. Die Permeanz der Membran wird deutlich geringer, wodurch die Membran ihre Eigenschaft als Nanofiltrationsmembran erhält. Bei der Trocknung ergibt sich bei der Verwendung der erfindungsgemäßen Unterstruktur zudem der Vorteil, dass die erfindungsgemäße Unterstruktur, die ein Polyaryletherketon (PAEK), vorzugsweise ein Polyetheretherketon (PEEK) enthält, im Gegensatz z. B. zu Polypropylen nicht schon bei ca. 120 bis 140 °C anfängt zu schmelzen. Dadurch können höhere Temperaturen eingesetzt werden und kürze Trockenzeiten realisiert werden. Außerdem hat das beispielhaft erwähnte Polypropylen den Nachteil, dass es auch weit unterhalb des Schmelzpunktes zur Kaltverformung neigt. Das bedeutet, dass Polypropylen sich auch bei Temperaturen verformen und es dadurch zu Problemen kommen kann. So müssen zum Beispiel im Falle einer Unterstruktur aus Polypropylen in einem kontinuierlichen Prozess von Rolle zu Rolle die Lenkwalzen penibel ausgerichtet werden, um eine Verstreckung der Unterstruktur und somit typischerweise auch der aufgetragenen Membran zu vermeiden.

Die erwähnte Trocknung im optionalen Schritt f) des erfindungsgemäßen Verfahrens kann kontinuierlich, beispielsweise in einem Bandtrockner, oder diskontinuierliche, beispielsweise in einem geeigneten Ofen, erfolgen. Die Trocknung kann dabei bei atmosphärischem Druck (gilt für kontinuierliche oder diskontinuierliche Trocknungsverfahren) oder bei Vakuum in einem Bereich von 10 mbar bis 300 mbar, vorzugsweise 50 mbar bis 250 mbar (gilt nur für kontinuierliche Trocknungsverfahren) durchgeführt wird.

Die Membrantrennung in Schritt b) wird vorzugsweise Temperatur von 25 bis 100°C, weiterhin bevorzugt 30 bis 80°C und besonders bevorzugt 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird. Optional ist auch ein Degasingschritt zwischen der Reaktionszone in Schritt a) und der Membrantrennung in Schritt b) vorhanden, um vorher leicht flüchtige Verbindungen wie Kohlenmonoxid und/oder restliche, über das Abgas nicht abgetrennte unreaktiven Verunreinigungen wie Stickstoff, Wasserstoff, Alkane und leichtsiedenden Nebenprodukte (beispielsweise die bereits genannten Ether) aus der Produktmischung zu entfernen. Dabei wird das Produktgemisch erst unter den Partialdruck der gelösten Komponenten wie Kohlenmonoxid entspannt, so dass diese ausgasen, um dann wieder den Druck zu erhöhen, der in der Membrantrennung vorgesehen ist.

Der Transmembrandruck (TMP) kann in Schritt b) 10 bis 60 bar, vorzugsweise 15 bis 55 bar, besonders bevorzugt 20 bis 50 bar betragen (relativ). Der permeatseitige Druck kann dabei oberhalb des atmosphärischen Drucks bis 15 bar, vorzugsweise 3 bis 7 bar betragen, woraus sich anhand des TMP dann der retentaseitige Druck ergibt. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur eingestellt wird, um eine Verdampfung nach dem Durchtritt durch die Membran zu vermeiden, da dadurch die gesamte Fahrweise instabil werden könnte. Gleiches gilt grundsätzlich auch für gelöste Komponenten wie Kohlenmonoxid, die optional durch den bereits genannten Degasingschritt entfernt werden können.

Die Ökonomie von Membrantrennverfahren kann sich stark nach der Lebensdauer der verwendeten Membranen bemessen, weshalb auch die Lebensdauer bzw. die Stabilität der Membran ein Kriterium für die Auswahl des geeigneten Membranmaterials sein kann. Dabei wird eine Mindestlebensdauer von etwa einem halben Jahr angenommen. Das kann insbesondere für Prozesse, wie dem vorliegenden Verfahren, bei denen das Produkt im Permeat angereichert wird, relevant werden, weil die erforderliche Membranfläche mit der Gesamtkapazität des Verfahrens steigt.

Daraus ergeben sich insbesondere drei Kriterien für die Lebensdauer bzw. die Stabilität der Membran, nämlich die technische Integrität der Membran, der Membranrückhalt (entspricht der Trennleistung der Membran, siehe nachfolgende Definition) und die Permeabilität der Membran (siehe nachfolgende Definition). Fehler im Hinblick auf die technische Integrität, wie die Auflösung der Membran, die Ausbildung von Fehlstellen oder Löchern oder eine deutliche Schrumpfung oder Vergrößerung der Membranfläche, können dabei auf den Rückhalt der Membran (Rückhalt sinkt) und die Permeabilität (Permeabilität steigt an) ausstrahlen und auch dafür sorgen, dass die Membran nicht mehr mit dem genannten Druck beaufschlagt werden kann und/oder sich der Transmembrandruck nicht einstellen kann.

In der Membrantechnik wird zur Charakterisierung der Durchlässigkeit bzw. der Trennleistung einer Membran der Rückhalt R der Membran hinsichtlich einer bestimmten Komponente des Stoffgemisches gemäß nachfolgender Formel (1) definiert:

$$R = 1 - w_{(I)P} / w_{(I)R} \qquad (1),$$

worin $w_{(I)P}$ für den Massenanteil der betrachteten Komponente im Permeat und $w_{(I)R}$ für den Massenanteil der betrachteten Komponente im Retentat der Membran steht. Der Rückhalt kann somit Werte von 0 bis 1 annehmen und wird deswegen gerne in % angegeben. Ein Rückhalt von 0 % bedeutet, dass die betrachtete Komponente ungehindert durch die Membran permeiert, sodass die Massenanteile der Komponenten im Retentat wie im Permeat gleich sind. Andererseits bedeutet ein Rückhalt von 100 %, dass die betrachtete Komponente vollständig von der Membran zurückgehalten wird, was sich technisch jedoch kaum realisieren lässt.

Neben dem Rückhalt ist auch die sogenannte Permeabilität der Membran für die Charakterisierung ihrer Durchlässigkeit gemäß der nachfolgenden Formel (2) maßgeblich:

$$P = m' / (A * TMP) \qquad (2),$$

worin m' für den Massenstrom des Permeats, A für die Fläche der Membran und TMP für den angelegten Transmembrandruck steht. Angegeben wird die Permeabilität in der Regel in der Einheit kg /(h * m² * bar). Die Permeabilität ist somit eine Kennzahl, die auf die Membranfläche und den eingestellten TMP normiert ist.

Im Hinblick auf die Charakterisierung der Stabilität einer Membran kann eine relative Änderung der Permeabilität $P_{Rel}$ gemäß der nachfolgenden Formel (3) definiert werden:

$$P_{Rel} = P_{t=x} / P_{t=0} \qquad (3),$$

worin $P_{t=x}$ für die Permeabilität zum Zeitpunkt t=x und $P_{t=0}$ für die ursprüngliche Permeabilität zum Zeitpunkt t=0 steht (möglich ist auch ein anderer zeitlicher Bezugspunkt, mit der Maßgabe, dass t=x > t=y).

Eine Membran kann weiterhin über die Selektivität bezüglich einer bestimmten Komponente charakterisiert werden. Die Selektivität bezeichnet das Verhältnis der Konzentrationen einer bestimmen Komponente i vor und nach der Membran. Die Selektivität berechnet sich nach der Formel (4):

$$S_i = C_{P\_i} / C_{F\_i} \qquad (4),$$

worin $C_{F\_i}$ für die Konzentration der betrachteten Komponente i im Feed zur Membran, hier das flüssige Produktgemisch, und $C_{P\_i}$ für die Konzentration der betrachteten Komponente i im Permeat steht.

Nach einer bevorzugten Ausführungsform der vorliegenden Erfindung weist das erfindungsgemäße Membranmaterial nach einem Einsatz im hier beschriebenen Membrantrennverfahren in Schritt b) von 300 Stunden die folgenden beiden Merkmale auf:

- der Membranrückhalt R für das homogene Katalysatorsystem beträgt mindestens 75%, vorzugsweise 85%, besonders bevorzugt 90% und verschlechtert sich um nicht mehr als 30 Prozentpunkte, vorzugsweise nicht mehr als 20 Prozentpunkte, besonders bevorzugt nicht mehr als 10 Prozentpunkte; oder
- die relative Permeabilität $P_{rel}$ weist einen Wert zwischen 30% und 300%, vorzugsweise zwischen 50% und 150%, belonders vorzugsweise zwischen 75% und 125% auf.

Die Membran sollte folglich nicht ihre technische Integrität verlieren. Beispiele für den Verlust der technischen Integrität sind die Auflösung der Membran, die Ausbildung von Fehlstellen/Löchern und/oder eine erhebliche Schrumpfung oder eine erhebliche Vergrößerung der Membranfläche. Das erfindungsgemäße Membranmaterial soll dabei insbesondere gegenüber der Anwesenheit von Säuren in einer Menge von 0,1 Gew.-% bis 5 Gew.-% stabil sein, nicht ihre technische Integrität verlieren und die oben genannten beiden Merkmale aufweisen.

Das Permeat aus der Membrantrennung (Schritt b)) wird im nachfolgenden Schritt c) einem Trennverfahren ausgewählt aus der Gruppe, bestehend aus einer thermischen Trennung, beispielsweise Destillation, einer Extraktion, einer Kristallisation oder einer weiteren Membrantrennung, unterworfen, um den in Schritt a) gebildeten Ester vom restlichen Permeat abzutrennen. Thermische Trennung im Sinne der vorliegenden Erfindung meint ein Trennverfahren, bei dem die Trennung anhand des Siedepunktes erfolgt. Das Trennverfahren ist vorzugsweise eine Destillation. Die entsprechenden Verfahrensbedingungen sind dem Fachmann bekannt.

Es kann vorkommen, dass bei dem in Schritt c) verwendeten Trennverfahren, insbesondere bei der Destillation, nicht nur der gebildete Ester aus dem Permeat abgetrennt wird, sondern auch die möglicherweise entstandenen Hochsieder, beispielsweise hochsiedende Nebenprodukte, die bei der Umsetzung in Schritt a) anfallen können. Um diese Hochsieder zu entfernen kann das erfindungsgemäße Verfahren einen Aufreinigungsschritt aufweisen, nämlich die Aufreinigung des gebildeten Esters durch Abtrennung des Esters von im Permeat enthaltenden Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Das in Schritt c) erhaltene zu großen Teilen vom in Schritt a) gebildeten Ester befreite Permeat, welches mindestens nicht umgesetzte Alkohole und/oder nicht umgesetzte Kohlenwasserstoffe enthält, wird in einer bevorzugten Ausführungsform einer Recyclekomponentenabtrennung unterworfen. Dabei werden die nicht umgesetzten Alkohole und/oder nicht umgesetzten Kohlenwasserstoffe vom restlichen Permeat, insbesondere den dort enthaltenen Leichtsiedern, mittels thermischer Trennung, Extraktion, Kristallisation oder Membrantrennung abgetrennt. Bevorzugt wird ein thermisches Trennverfahren, besonders bevorzugt eine weitere Destillation eingesetzt, um die nicht umgesetzten Alkohole und/oder nicht umgesetzten Kohlenwasserstoffe vom restlichen Permeat abzutrennen. Die Verfahrensbedingungen sind dem Fachmann bekannt. Die dabei erhaltenen nicht umgesetzten Alkohole und/oder die nicht umgesetzten Kohlenwasserstoffe können dann in die Reaktionszone zurückgeführt werden.

Das Verfahren ist gemäß einer besonders bevorzugten Ausführungsform der vorliegenden Erfindung ein Verfahren zur Herstellung eines Esters durch Carbonylierung eines C4- bis C12 Kohlenwasserstoffs, wobei das Verfahren die folgenden Schritte umfasst:

a) Umsetzen (Carbonylieren) eines n-Alkens und/oder iso-Alkens mit 4 bis 12 Kohlenstoffatomen mit Kohlenmonoxid und mit einem Alkohol, ausgewählt aus der Gruppe, bestehend aus Methanol, Ethanol, 1-Propanol, 1-Butanol, 1-Pentanol, 1-Hexanol, 2-Propanol, tert-Butanol und 3-Pentanol, in Gegenwart eines homogenen Katalysatorsystems, welches Pd oder Pt oder eine Verbindung davon, einen phosphorhaltigen Liganden und eine Säure als Co-Katalysator umfasst, in einer Reaktionszone unter Erhalt eines Produktgemisches, welches zumindest den durch die Umsetzung gebildeten Ester, das homogene Katalysatorsystem, Leichtsieder, Hochsieder und nicht umgesetzte Alkohole umfasst, wobei die Umsetzung bei einer Temperatur von 80 bis 120 °C und einem Druck von 10 bis 40 bar durchgeführt wird;

b) Durchführen einer Membrantrennung zur Abtrennung des homogenen Katalysatorsystems aus dem Produktgemisch, wodurch das homogene Katalysatorsystem und zusätzlich nicht umgesetzter Kohlenwasserstoff und/oder nicht umgesetzter Alkohol, vorzugsweise nicht umgesetzter Alkohol im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht, und Rückführung des Retentats in die Reaktionszone;

c) Abtrennen des in Schritt a) gebildeten Esters aus dem Permeat mittels thermischer Trennung oder Extraktion vorzugsweise mittels Destillation.

Der nach dem erfindungsgemäßen Verfahren gebildete Ester kann in zwei weiteren nachfolgenden Verfahrensschritten d) und e) umgeestert werden. Dabei wird der Teil des Esters, der dem in Schritt a) eingesetzten ersten Alkohol entspricht, durch einen zweiten Alkohol ausgetauscht. Diese Umesterung wird nach dem oben genannten Schritt c), optional nach dem möglichen Aufreinigungsschritt, durchgeführt und umfasst die folgenden Schritte:

d) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs, welches zumindest den Ester mit dem zweiten Alkohol, den abgespaltenen ersten Alkohol und nicht umgesetzte zweite Alkohole umfasst;

e) Abtrennen des gebildeten Esters mit dem zweiten Alkohol vom restlichen zweiten Produktgemisch und insbesondere vom abgespaltenen ersten Alkohol mittels eines oder mehrerer Trennschritte, ausgewählt aus thermischer Trennung, Extraktion, Kristallisation oder Membrantrennung, vorzugsweise durch thermische Trennung und/oder mittels Membrantrennung, besonders bevorzugt mittels eines oder mehrerer Destillationsschritte und Rückführung des abgespaltenen ersten Alkohols in die Reaktionszone aus Schritt a), sowie Rückführung des nicht umgesetzten zweiten Alkohols in die zweite Reaktionszone.

In Schritt d) erfolgt die eigentliche Umesterung, also die Abspaltung des eigentlich in Schritt a) angebundenen ersten Alkohols und die Anbindung des zweiten Alkohols. Dazu wird der in Schritt a) gebildete Ester in einer Reaktionszone mit einem zweiten Alkohol, der sich vom ersten Alkohol unterscheidet, umgesetzt. In einer besonders bevorzugten Ausführungsform ist der bei der Umesterung eingesetzte zweite Alkohol im Vergleich zu dem in Schritt a) eingesetzten ersten Alkohol ein höhersiedender Alkohol. Der zweite Alkohol wird bei der Umesterung vorzugsweise im Überschuss zugegeben, um die Umesterungsreaktion zu begünstigen.

Der bei der Umesterung in Schritt d) eingesetzte zweite Alkohol ist vorzugsweise ein Mono- oder Polyalkohol (zwei oder mehr OH-Gruppen) mit 1 bis 50 Kohlenstoffatomen, weiterhin bevorzugt mit 1 bis 15 Kohlenstoffatomen, besonders bevorzugt mit 1 bis 10 Kohlenstoffatomen oder eine Mischung aus zwei oder mehr Mono- und/oder Polyalkoholen, mit der Maßgabe, dass der in Schritt a) eingesetzte erste Alkohol und der zweite Alkohol nicht identisch sind. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol, Pentaerythrit, Neopentylglykol, Trimethylolpropan, Dipentaerythriol oder Mischungen daraus, vorzugsweise Ethanol, 1-Propanol, 1-Butanol, 1-Pentanol, 1-Hexanol, 2-Propanol, tert-Butanol, 3-Pentanol, 2Propylheptanol.

Die Umesterung in Schritt d) wird vorzugsweise säure- oder basenkatalysiert durchgeführt. Als Säuren können Brönsted- oder Lewis-Säuren eingesetzt werden.

Geeignete Brönsted-Säuren für die Umesterung in Schritt d) sind Perchlorsäure, Schwefelsäure, Phosphorsäure, Methylphosphonsäure oder eine Sulfonsäure, beispielsweise Methansulfonsäure, Trifluormethansulfonsäure, tert-Butansulfonsäure, p-Toluolsulfonsäure(pTSA), 2-Hydroxypropan-2-sulfonsäure, 2,4,6-Trimethylbenzolsulfonsäure oder Dodecylsulfonsäure. Vorzugsweise werden Schwefelsäure oder eine Sulfonsäure als Brönsted-Säure eingesetzt, besonders bevorzugt Schwefelsäure. Es können auch Metall oder deren Verbindungen eingesetzt werden, beispielsweise Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Geeignete Lewis-Säuren für die Umesterung in Schritt d) sind Titan(IV)-isopropoxid, $Bu_2SnO$, $BuSn(O)OH$ oder Aluminiumtriflat. Bevorzugt eingesetzte Lewis-Säuren sind Titan(IV)-isopropoxid, und Aluminiumtriflat

Geeignete Basen für die Umesterung in Schritt d) sind Alkalimetalle, Alkalialkoxide, Alkali- oder Erdalkaliacetate oder -oxide, Alkali- oder Erdalkalialkoholate, wie NaEtOH oder MgEtOH oder Alkalicarbonate, wie $K_2CO_3$ oder $Cs_2CO_3$. Es können aber auch basische Ionenaustauscher oder NaOH eingesetzt werden. Bevorzugt werden Na- oder Mg-Alkoholate wie NaEtOH oder MgEtOH eingesetzt.

Die säurekatalysierten Umesterung wird vorzugsweise bei einer Temperatur von 60 bis 220 °C, weiterhin bevorzugt von 100 bis 210 °C und besonders bevorzugt bei 130 bis 200 °C durchgeführt. Die Reaktion findet vorzugsweise oberhalb des Siedepunktes des abzuspaltenden ersten Alkohols statt, um den abgespaltenen ersten Alkohol direkt aus dem Reaktionsgemisch zu entfernen und somit eine Gleichgewichtsverlagerung zur Produktseite zu begünstigen. Der zweite Alkohol wird dabei vorzugsweise in einem signifikanten Überschuss, beispielsweise 30 : 1, zum in Schritt a) gebildeten Ester hinzugegeben. Die basenkatalysierte Umesterung findet vorzugsweise bei einer Temperatur von 20 bis 100 °C statt.

Durch die beschriebe Umesterung wird ein zweites Produktgemisch erhalten, welches zumindest den Ester mit dem zweiten Alkohol, den abgespaltenen ersten Alkohol und nicht umgesetzte zweite Alkohole umfasst.

Der in Schritt d) gebildete zweite Ester wird im nachfolgenden Schritt e) vom restlichen zweiten Produktgemisch abgetrennt. Die Abtrennung wird mittels thermischer Trennung, vorzugsweise Destillation, und/oder mittels Membrantrennung, insbesondere mit den oben beschriebenen Membranmaterialien, durchgeführt. Die entsprechenden Verfahrensbedingungen sind dem Fachmann bekannt.

Es kann vorkommen, dass bei dem in Schritt e) verwendeten Trennverfahren, insbesondere bei der Destillation, nicht nur der gebildete Ester vom restlichen zweiten Produktgemisch abgetrennt wird, sondern auch möglicherweise gebildete Hochsieder, beispielsweise hochsiedende Nebenprodukte, die bei der Umsetzung in Schritt c1) anfallen können. Um diese Hochsieder zu entfernen kann das erfindungsgemäße Verfahren einen Aufreinigungsschritt aufweisen, nämlich die Aufreinigung des in Schritt c1) gebildeten Esters durch Abtrennung des Esters von den vorhandenen Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Die erfindungsgemäß erhaltenen Ester können als Edukte zur Herstellung weiterer Produkte dienen. Wichtige Reaktionen der erhaltenen Ester sind die Hydrierung zur Bildung von Alkoholen, die Hydrolyse bzw. Verseifung zur Bildung von Carbonsäuren bzw. Carbonsäuresalzen oder die Aminolyse zur Bildung von Amiden.

**Patentansprüche**

1.  Verfahren zur Herstellung eines Esters durch Carbonylierung eines C2- bis C20-Kohlenwasserstoffs, wobei das Verfahren die folgenden Schritte umfasst:

    a) Umsetzen (Carbonylieren) eines C2- bis C16-Kohlenwasserstoffs mit mindestens einer olefinischen Doppelbindung mit Kohlenmonoxid und mit einem Alkohol, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher, wenn es sich um einen Monoalkohol handelt, in einem Molverhältnis zum C2- bis C16-Kohlenwasserstoff (Alkohol: C2- bis C16-Kohlenwasserstoff) von 2 bis 20 eingesetzt wird oder welcher, wenn es sich um einen Polyalkohol handelt, in einem Molverhältnis zum eingesetzten Kohlenwasserstoff (Kohlenwasserstoff: Polyalkohol) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches, welches zumindest den durch die Umsetzung gebildeten Ester, das homogene Katalysatorsystem, Leichtsieder, Hochsieder und nicht umgesetzte Alkohole umfasst;
    b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol, vorzugsweise nicht umgesetzter Alkohol im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht, und Rückführung des Retentats in die Reaktionszone;
    c) Aufarbeitung des Permeat mittels eines oder mehrerer Trennschritte, ausgewählt aus thermischer Trennung, Extraktion, Kristallisation oder Membrantrennung, vorzugsweise mittels eines oder mehrerer Destillationsschritte, zur Abtrennung des in Schritt a) gebildeten Esters und zur Abtrennung des nicht umgesetzten C2- bis C16-Kohlenwasserstoffs mit mindestens einer olefinischen Doppelbindung und/oder des nicht umgesetzten Alkohols und Rückführung des nicht umgesetzten C2- bis C16-Kohlenwasserstoffs mit mindestens einer olefinischen Doppelbindung und/oder des nicht umgesetzten Alkohols in die Reaktionszone von Schritt a).

2.  Verfahren nach Anspruch 1, wobei die Unterstruktur der bei der Membrantrennung eingesetzten Membran zu mindestens 90 Gew.-% aus dem Polyaryletherketon (PAEK), vorzugsweise zu mindestens 99 Gew.-% aus dem Polyaryletherketon (PAEK) und besonders bevorzugt ausschließlich aus dem Polyaryletherketon (PAEK) besteht.

3.  Verfahren nach Anspruch 2, wobei die Unterstruktur der bei der Membrantrennung eingesetzten Membran zu mindestens 90 Gew.-% aus einem Polyetheretherketon (PEEK), vorzugsweise zu mindestens 99 Gew.-% aus einem Polyetheretherketon (PEEK)und besonders bevorzugt ausschließlich aus dem einem Polyetheretherketon (PEEK) besteht.

4.  Verfahren nach Anspruch 3, wobei die Unterstruktur der bei der Membrantrennung eingesetzten Membran ausschließlich aus einem Polyetheretherketon (PEEK) besteht.

5.  Verfahren nach einem der vorhergehenden Ansprüche, wobei die trennaktive Schicht der bei der Membrantrennung eingesetzten Membran aus einem Polyetheretherketon (PEEK) besteht.

6.  Verfahren nach Anspruch 1 oder 2, wobei die trennaktive Schicht und/oder die Unterstruktur aus einem Polyaryletherketon (PAEK) mit einem Sulfonierungsgrad von weniger als 20%, besonders bevorzugt weniger als 10% besteht.

7.  Verfahren nach einem der Ansprüche 3 bis 5, wobei die trennaktive Schicht und/oder die Unterstruktur ein Polyetheretherketon (PEEK) mit einem Sulfonierungsgrad von weniger als 20%, besonders bevorzugt weniger als 10% umfassen oder daraus bestehen.

8.  Verfahren nach einem der vorhergehenden Ansprüche, wobei die Unterstruktur der Membran eine mindestens zweifach höhere Permeanz, vorzugsweise eine mindestens 10-fach höhere Permeanz, besonders bevorzugt eine mindestens 100-fach höhere Permeanz aufweist als die trennaktive Schicht

9.  Verfahren nach einem der vorhergehenden Ansprüche, wobei der in Schritt a) eingesetzte Alkohol ein Mono- oder Polyalkohol (zwei oder mehr OH-Gruppen) mit 1 bis 15 Kohlenstoffatomen oder eine Mischung aus zwei oder mehr Mono- und/oder Polyalkoholen ist.

10. Verfahren nach Anspruch 9, wobei der in Schritt a) eingesetzte Alkohol aus der Gruppe, bestehend aus Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol und Mischungen daraus, ausgewählt wird.

11. Verfahren nach einem der vorhergehenden Ansprüche, wobei das Metall der Gruppe 8 bis 10 des Periodensystems der Elemente oder eine Verbindung davon des homogenen Katalysatorsystems in Schritt a) Palladium oder eine Verbindung davon ist.

12. Verfahren nach einem der vorhergehenden Ansprüche, wobei die Reaktionszone für die Umsetzung in Schritt a) mindestens einen Reaktor aufweist.

13. Verfahren nach einem der vorhergehenden Ansprüche, wobei die Säure des Katalysatorsystems in Schritt a) eine Brönsted-Säure mit einem pKs $\leq$ 5, vorzugsweise mit einem pKs $\leq$ 3 oder eine Lewis-Säure mit einem LAU-Wert von mehr als 25, vorzugsweise mit einem LAU-Wert von 29 ist.

14. Verfahren nach einem der vorhergehenden Ansprüche, wobei die Säure des Katalysatorsystems in Schritt a) eine Brönsted-Säure oder eine Lewis-Säure ist, wobei die Brönsted-Säure Perchlorsäure, Schwefelsäure, Phosphorsäure, Methylphosphonsäure oder eine Sulfonsäure ist und wobei die Lewis-Säure Aluminiumtriflat, Aluminiumchlorid, Aluminiumhydrid, Trimethylaluminium, Tris(pentafluorophenyl)boran, Bortrifluorid, Bortrichlorid oder eine Mischungen davon ist.

15. Verfahren nach einem der vorhergehenden Ansprüche, wobei das Verfahren nach der Abtrennung in Schritt c) die folgenden weiteren Schritte aufweist:

d) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs, welches zumindest den Ester mit dem zweiten Alkohol, den abgespaltenen ersten Alkohol und nicht umgesetzte zweite Alkohole umfasst, wobei der zweite Alkohol in äquimolarer Menge oder im Überschuss eingesetzt wird.

e) Abtrennen des gebildeten Esters mit dem zweiten Alkohol vom restlichen zweiten Produktgemisch und insbesondere vom abgespaltenen ersten Alkohol mittels eines oder mehrerer Trennschritte, ausgewählt aus thermischer Trennung, Extraktion, Kristallisation oder Membrantrennung, vorzugsweise durch thermische Trennung und/oder mittels Membrantrennung und Rückführung des abgespaltenen ersten Alkohols in die Reaktionszone aus Schritt a), sowie Rückführung des nicht umgesetzten zweiten Alkohols in die zweite Reaktionszone.

Europäisches
Patentamt

European
Patent Office

Office européen
des brevets

# EUROPÄISCHER RECHERCHENBERICHT

**Nummer der Anmeldung**

**EP 21 21 5862**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/392064 A1 (KUCMIERCZYK PETER [DE] ET AL) 17. Dezember 2020 (2020-12-17) * Anspruch 1 * ----- | 1-15 | INV. B01D61/02 B01D69/12 B01D71/52 B01J31/00 B01J31/40 |
| A | US 2020/392062 A1 (KUCMIERCZYK PETER [DE] ET AL) 17. Dezember 2020 (2020-12-17) * Anspruch 1 * ----- | 1-15 | |
| A | US 2020/392057 A1 (KUCMIERCZYK PETER [DE] ET AL) 17. Dezember 2020 (2020-12-17) * Anspruch 1 * ----- | 1-15 | |
| A | US 2020/391194 A1 (KUCMIERCZYK PETER [DE] ET AL) 17. Dezember 2020 (2020-12-17) * Anspruch 1 * ----- | 1-15 | |
| A | US 2017/007963 A1 (LIVINGSTON ANDREW GUY [GB] ET AL) 12. Januar 2017 (2017-01-12) * Anspruch 1 * ----- | 1-15 | |

**RECHERCHIERTE SACHGEBIETE (IPC)**

B01D
B01J

Der vorliegende Recherchenbericht wurde für alle Patentansprüche erstellt

| Recherchenort | Abschlußdatum der Recherche | Prüfer |
|---|---|---|
| München | 8. Juni 2022 | Hoyer, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.............................................................................
& : Mitglied der gleichen Patentfamilie, übereinstimmendes Dokument

EPO FORM 1503 03.82 (P04C03)

**EP 4 197 623 A1**

## ANHANG ZUM EUROPÄISCHEN RECHERCHENBERICHT
## ÜBER DIE EUROPÄISCHE PATENTANMELDUNG NR.

EP 21 21 5862

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-06-2022

| Im Recherchenbericht angeführtes Patentdokument | Datum der Veröffentlichung | Mitglied(er) der Patentfamilie | Datum der Veröffentlichung |
|---|---|---|---|
| US 2020392064 A1 | 17-12-2020 | CN 112079718 A | 15-12-2020 |
| | | EP 3750620 A1 | 16-12-2020 |
| | | ES 2912561 T3 | 26-05-2022 |
| | | JP 2020200328 A | 17-12-2020 |
| | | KR 20200142468 A | 22-12-2020 |
| | | SG 10202005404Y A | 28-01-2021 |
| | | TW 202110783 A | 16-03-2021 |
| | | US 2020392064 A1 | 17-12-2020 |
| | | ZA 202003461 B | 28-07-2021 |
| US 2020392062 A1 | 17-12-2020 | CN 112321431 A | 05-02-2021 |
| | | EP 3750865 A1 | 16-12-2020 |
| | | JP 2020200327 A | 17-12-2020 |
| | | KR 20200142466 A | 22-12-2020 |
| | | SG 10202005402U A | 28-01-2021 |
| | | TW 202100503 A | 01-01-2021 |
| | | US 2020392062 A1 | 17-12-2020 |
| | | ZA 202003436 B | 28-07-2021 |
| US 2020392057 A1 | 17-12-2020 | CN 112125801 A | 25-12-2020 |
| | | EP 3750866 A1 | 16-12-2020 |
| | | ES 2911512 T3 | 19-05-2022 |
| | | JP 2020200326 A | 17-12-2020 |
| | | KR 20200142467 A | 22-12-2020 |
| | | PL 3750866 T3 | 23-05-2022 |
| | | SG 10202005401W A | 28-01-2021 |
| | | TW 202110782 A | 16-03-2021 |
| | | US 2020392057 A1 | 17-12-2020 |
| | | ZA 202003435 B | 28-07-2021 |
| US 2020391194 A1 | 17-12-2020 | CN 112246103 A | 22-01-2021 |
| | | EP 3750627 A1 | 16-12-2020 |
| | | US 2020391194 A1 | 17-12-2020 |
| US 2017007963 A1 | 12-01-2017 | EP 3099400 A1 | 07-12-2016 |
| | | US 2017007963 A1 | 12-01-2017 |
| | | WO 2015110843 A1 | 30-07-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013107902 A1

- EP 3121184 A2

**In der Beschreibung aufgeführte Nicht-Patentliteratur**

- **JR GAFFEN et al.** *Chem,* vol. 5 (6), 1567-1583